(19) Europäisches Patentamt
European Patent Office
Office européen des brevets

(11) EP 0 571 819 B1

(12) # EUROPÄISCHE PATENTSCHRIFT

(45) Veröffentlichungstag und Bekanntmachung des Hinweises auf die Patenterteilung:
**11.11.1998 Patentblatt 1998/46**

(51) Int Cl.[6]: **C07F 9/50**, C07C 45/50, B01J 31/24

(21) Anmeldenummer: **93107701.0**

(22) Anmeldetag: **12.05.1993**

(54) **Sulfonierte 2,2'-Bis(diphenylphosphinomethyl)-1,1'-binaphthaline, Verfahren zu ihrer Herstellung und ihre Verwendung in einem Verfahren zur Hydroformylierung von olefinisch ungesättigten Verbindungen**

Sulfonated 2,2'-bis(diphenylphosphinomethyl)-1,1'-binaphthalenes, process for their preparation and their use in a process for the hydroformylation of olefinic unsaturated compounds

2,2'-Bis(diphénylphosphinométhyl)-1,1'-binaphthalènes sulfonés, procédé de leur préparation et leur utilisation dans un procédé pour l'hydroformylation de composés oléfiniques non saturés

(84) Benannte Vertragsstaaten:
**AT BE CH DE DK ES FR GB IT LI NL SE**

(30) Priorität: **29.05.1992 DE 4217803**
**28.12.1992 DE 4244274**

(43) Veröffentlichungstag der Anmeldung:
**01.12.1993 Patentblatt 1993/48**

(73) Patentinhaber: **Celanese GmbH**
**60439 Frankfurt (DE)**

(72) Erfinder:
- **Herrmann, Wolfgang A., Prof. Dr. Dipl.-Chem.**
**W-8051 Giggenhausen (DE)**
- **Manetsberger, Rainer Dr.**
**D-82407 Wielenbach (DE)**
- **Bahrmann, Helmut, Dr. Dipl.-Chem.**
**W-4236 Hamminkeln 3 (DE)**
- **Kohlpaintner, Christian, Dipl.-Chem.**
**W-8209 Stephanskirchen (DE)**
- **Lappe, Peter, Dr. Dipl.-Chem.**
**W-4220 Dinslaken (DE)**

(56) Entgegenhaltungen:
**EP-A- 0 279 018**
**EP-A- 0 376 856**
**EP-A- 0 491 239**
**DE-A- 2 627 354**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 571 819 B1

**Beschreibung**

Die Erfindung betrifft neue sulfonierte Diphosphine und ihre Herstellung. Sie bilden mit Metallen der VIII. Gruppe des Periodensystems der Elemente Komplexverbindungen, die als Katalysatoren Anwendung finden können.

Komplexverbindungen, die als Zentralatom ein Metall der VIII. Gruppe des Periodensystems der Elemente und als Liganden P(III)-verbindungen wie Phosphine oder Phosphite und daneben gegebenenfalls noch weitere, zur Komplexbildung befähigte Gruppen enthalten, haben in jüngster Zeit als Katalysatoren zunehmende Bedeutung gewonnen. So erfolgt die technisch in großem Umfang ausgeübte Reaktion von Olefinen mit Kohlenmonoxid und Wasserstoff zu Aldehyden (Hydroformylierung) in Gegenwart von Katalysatorsystemen, die aus Rhodium und Triphenylphosphin bestehen. Auch zur Umsetzung von Methanol mit Synthesegas zu höheren Alkoholen, insbesondere Ethanol und Propanol (Homologisierung), haben sich Katalysatoren auf Basis von Phosphine enthaltenden Komplexverbindungen bewährt. Zumeist liegen in den genannten Fällen die Liganden im Überschuß vor, so daß das Katalysatorsystem aus Komplexverbindung und freiem Ligand besteht. Entsprechend der Löslichkeit dieser Systeme in organischen Medien erfolgt die Umsetzung in homogener Phase.

Aus EP-A-0 376 856 sind Diphosphin-Verbindungen wie z.B. 2,2'-Bis(diphenylphosphinomethyl)-1,1'-binaphthalin bekannt, die gemäß EP-A-0 279 018 auch als Liganden in Rhodium-haltigen Katalysatorsystemen zur Hydroformylierung von Olefinen in homogener Phase eingesetzt werden können.

Statt in homogener Phase kann man die Umsetzung auch in heterogener Phase durchführen. Diese Verfahrensvariante ist besonders zweckmäßig, weil sie einen Weg eröffnet, den in Wasser gelösten Katalysator einfach und schonend von dem in Wasser nicht löslichen Reaktionsprodukt abzutrennen. Nach diesem Prinzip arbeitet z.B. das in der DE 26 27 354 C2 beschriebene Hydroformylierungsverfahren. Als Katalysator gelangt das System Rhodium/Na-Triphenylphosphintrisulfonat zum Einsatz.

Neben Monophosphinen werden auch Diphosphine als Bestandteil von Katalysatorsystemen eingesetzt, deren andere Komponente ein Metall der VIII. Gruppe des Periodensystems ist. Beispielsweise ist die Herstellung von Aldehyden durch Umsetzung von Monoolefinen, nicht konjugierten Polyolefinen, Cycloolefinen oder Derivaten dieser Verbindungsklassen mit Kohlenmonoxid und Wasserstoff in Gegenwart von Rhodium/Diphosphin-Katalysatoren Gegenstand der DE-A 4040 315. Als Diphosphine finden in diesem Verfahren sulfonierte 2,2'-Bis(diphenylphosphinomethyl)biphenyle oder 2-(Diphenylphosphinomethyl)-1-[2-(diphenylphosphinomethyl)phenyl]naphthaline Anwendung. Zusammen mit dem Rhodium ergeben sie Katalysatoren, die sich gegenüber den bekannten Rhodium/Monophosphin-Systemen durch erhöhte Wirksamkeit auszeichnen.

Die Annahme, daß die Zusammensetzung des unter ihrem Einfluß entstandenen Produktes und die Aktivität von Rhodiumkomplex-Katalysatoren von der chemischen Eigenart der Liganden abhängt, führt zu der Aufgabe, neue Liganden zu entwickeln. Sie sollen die Möglichkeit eröffnen, den Reaktionsablauf zu variieren, so daß bestimmte Produkte bevorzugt, gegebenenfalls sogar ausschließlich, entstehen. Zudem wird gefordert, daß die Liganden und die von ihnen gebildeten Komplexverbindungen in Wasser löslich sind, um die vorstehend beschriebenen Vorteile der Hydroformylierung in einem Zweiphasensystem zu nutzen.

Die Erfindung stellt solche Liganden bereit. Sie betrifft neue sulfonierte 2,2'-Bis(diphenylphosphinomethyl)-1,1'-binaphthaline der allgemeinen Formel

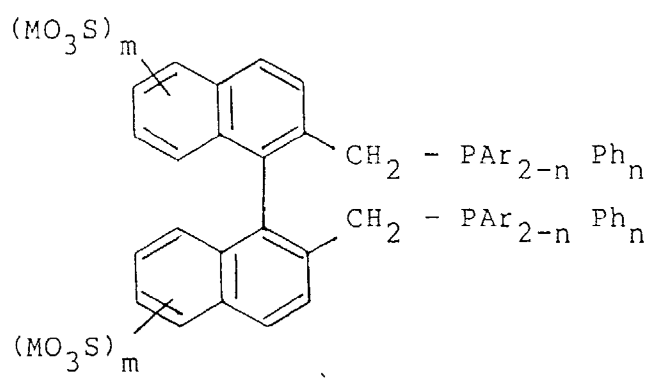

in der Ar für m-$C_6H_4$-$SO_3M$, M für Wasserstoff, für Ammonium, ein einwertiges Metall oder das Äquivalent eines mehrwertigen Metalls, Ph für den Phenylrest steht, m gleich oder verschieden ist und den Wert 1 oder 2 hat und n gleich oder verschieden ist und 0,1 oder 2 bedeutet.

Zur Herstellung der neuen Verbindungen geht man von 2,2'-Bis(diphenylphosphinomethyl)-1,1'-binaphthalin als Grundkörper aus. Diese Verbindung erhält man in einer mehrstufigen Synthese durch reduktive Dimerisierung von

1-Brom-2-methylnaphtalin mit Magnesium zu 2,2'-Dimethyl-1,1'-binaphthalin, Umsetzung des Binaphthalins mit Butyllithium zur Dilithiumverbindung und Reaktion der Dilithiumverbindung mit Chlordiphenylphosphin. Statt 2,2'-Dimethyl-1,1'-binaphthalin mit Butyllithium zu metallieren, kann man es auch mit N-Bromsuccinimid in das 2,2'-Bis(brommethyl)-1,1'-binaphthalin überführen. Durch Umsetzung mit Diphenylphosphinigsäureester erhält man aus der Dibromverbindung 2,2'-Bis(diphenylphosphinylmethyl)-1,1'-binaphthalin, das mit Trichlorsilan zum 2,2'-Bis(diphosphinomethyl)-1,1'-binaphthalin reduziert wird. Zur Einführung von Sulfonsäuregruppen in den Binaphthylrest und in die Phenylreste behandelt man das Diphosphin mit überschüssigem Schwefeltrioxid in Form von Oleum als Sulfonierungsmittel.

Maßgebend für den erzielbaren Sulfonierungsgrad sind insbesondere die $SO_3$-Konzentration im Oleum, die Reaktionstemperatur und die Reaktionszeit. Die genannten Parameter beeinflussen sich gegenseitig.

Es hat sich bewährt Oleum einzusetzen, das 10 bis 65 aber auch mehr Gew.-% Schwefeltrioxid enthält. Das Sulfonierungsmittel ist, bezogen auf das Diphosphin, im Überschuß anzuwenden. Zweckmäßig setzt man je mol 2,2'-Bis (diphenylphosphinomethyl)-1,1'-binaphthalin 25 bis 80, vorzugsweise 40 bis 70 mol $SO_3$ ein. Oleum mit freiem $SO_3$ in hoher Konzentration, d.h. einem Anteil von etwa 40 bis 65 und mehr Gew.-% führt zu Produkten, die mindestens vier $SO_3H$-Gruppen enthalten und daher in Wasser ausgezeichnet löslich sind. Konzentrationen von freiem $SO_3$ in Oleum, die niedriger als etwa 40 Gew.-% sind, ergeben geringer sulfonierte Produkte, d.h. Diphosphine, die in Wasser nur begrenzt löslich sind.

Die Reaktionstemperatur beträgt 0 bis 25°C, vorzugsweise 0 bis 10°C. Grundsätzlich können auch höhere Temperaturen angewandt werden, sie fördern jedoch die Oxidation der Diphosphine zu Phosphinoxiden wesentlich stärker als die Sulfonierung, so daß insgesamt die Ausbeute an sulfonierten Phosphinen abnimmt. Daher empfiehlt es sich auch nicht, niedrige Konzentrationen an freiem $SO_3$ durch Erhöhung der Reaktionstemperatur zu kompensieren. Dagegen gelingt es, den Sulfonierungsgrad des Diphosphins durch die Reaktionszeit zu beeinflussen. Längere Reaktionszeiten führen zu höher sulfonierten Verbindungen als kürzere. Im allgemeinen erfordert die Umsetzung in den genannten Temperaturbereichen 10 bis 60, vorzugsweise 15 bis 48 h. Dieser Zeitrahmen gilt insbesondere bei Anwendung von Oleum, das etwa 40 und mehr Gew.-% freies Oleum enthält. Niedriger konzentriertes Oleum führt auch bei langen Reaktionszeiten nur zu partiell sulfonierten Verbindungen, überdies läßt sich die zunehmende Bildung von Oxidationsprodukten nicht völlig vermeiden. Zweckmäßig führt man daher die Sulfonierung mit höher konzentriertem Oleum durch und steuert den Sulfonierungsgrad über die Reaktionszeit.

Als Lösungsmittel für die zu sulfonierende Ausgangsverbindung 2,2'-Bis(diphenylphosphinomethyl)-1,1'-binaphthalin hat sich konzentrierte Schwefelsäure bewährt. Diese Lösung kann anteilsweise in Oleum eingetragen oder anteilsweise mit Oleum versetzt werden. Es empfiehlt sich, das Reaktionsgemisch intensiv zu rühren, gut zu kühlen und die Reaktanten langsam und in kleinen Anteilen zusammenzuführen, so daß die Reaktionswärme ohne Mühe abgeführt werden kann. Dadurch erreicht man, daß die Sulfonierung ni-cht unkontrolliert abläuft, sondern in den Binaphthylrest und die Phenylreste nacheinander $SO_3H$-Gruppen eintreten. Überdies wird eine Oxidation der Phosphorverbindung wirksam unterbunden. Nach Zusatz des gesamten Sulfonierungsmittels bzw. Diphosphins kann die Nachreaktion bei Raumtemperatur, d.h. bei etwa 20 bis 25°C und im wesentlichen ohne äußere Kühlung erfolgen. Es ist jedoch zweckmäßig, das Reaktionsgemisch auch in diesem Stadium zu rühren, um gegebenenfalls noch auftretende Reaktionswärme gleichmäßig zu verteilen und ohne Verzögerung abführen zu können.

Im Anschluß an die Sulfonierung wird die Reaktionslösung hydrolysiert. Bei diesem Verfahrensschritt ist darauf zu achten, daß eine Temperatur von etwa 30°C nicht überschritten wird, vorteilhaft ist es, Temperaturen im Bereich von 15 bis 25°C einzuhalten. Es empfiehlt sich daher, das Umsetzungsgemisch vorsichtig auf Eis zu geben oder die Hydrolyse mit Eis oder Eiswasser durchzuführen und für intensive Außenkühlung zu sorgen. Die verdünnte, im wesentlichen die unterschiedlichen Sulfonierungsstufen des 2,2'-Bis(diphenylphosphinomethyl)-1,1'-binaphthalins und Schwefelsäure enthaltende Lösung wird in einem weiteren Verfahrensschritt aufgearbeitet.

Hierzu neutralisiert man die schwefelsaure Lösung mit einem alkalischen Reagenz wie Alkalihydroxid oder Alkalicarbonat, bevorzugt Natriumhydroxid. Um das Volumen des Reaktionsgemisches nicht unnötig zu vergrößern und das sich bildende Alkalisulfat weitgehend abzuscheiden, wendet man das Neutralisationsmittel als hochkonzentrierte Lösung oder in ungelöster fester Form an, z.B. Ätznatron-Schuppen oder -Granalien.

Die Hauptmenge des Alkalisulfats kann man aus der Lösung aufgrund seiner geringeren Löslichkeit bei niederen Temperaturen durch Abkühlen entfernen. Die geeigneten Temperaturen hängen von der Konzentration des Sulfats in der Lösung und dem Temperaturverlauf seiner Löslichkeit ab. Die günstigsten Bedingungen sind daher von Fall zu Fall durch Versuche zu bestimmen. Die Sulfatabtrennung kann in einer oder auch in mehreren Stufen erfolgen, es hat sich als zweckmäßig erwiesen, die Kristallisation in zwei Stufen vorzunehmen.

Nach der Abscheidung des Alkalisulfats engt man die Lösung vorzugsweise im Vakuum einer Ölpumpe zur Trockne ein und extrahiert aus dem Kristallbrei die Diphosphine unterschiedlichen Sulfonierungsgrades in Form der Alkalisalze. Geeignete Extraktionsmittel sind z.B. Gemische aus niederen Alkoholen, d.h. Alkoholen mit bis zu 5 Kohlenstoffatomen im Molekül, wie Methanol, Ethanol, Propanol mit Wasser. Man führt die Extraktion nach konventionellen Verfahren in einer oder in mehreren Stufen, vorzugsweise zwei bis vier Stufen durch. Die Extrakte werden vereinigt und zur Trockne eingeengt.

Ebenso hat es sich bewährt, die schwefelsaure Lösung mit einer Mischung aus einem in Wasser unlöslichen Amin und einem organischen Lösungsmittel umzusetzen und die Sulfonate als Aminsalze zu extrahieren. Geeignet sind Amine mit 10 bis 60, vorzugsweise 13 bis 36 Kohlenstoffatomen, z.B. Methyl-dioctylamin, Tri-n-octylamin, Triisooctylamin, Tri-2-ethylhexylamin, Tridodecylamin, vorzugsweise Triisooctylamin. Als organisches Lösungsmittel werden mit Erfolg aliphatische und aromatische Kohlenwasserstoffe oder Kohlenwasserstoffgemische, z.B. Toluol oder kerosinähnliche Gemische, insbesondere Toluol, verwendet.

Je Äquivalent Sulfonsäure setzt man 0,5 bis 1,5 mol, vorzugsweise 0,8 bis 1,2 mol Amin ein. Nach intensivem Mischen der schwefelsauren und der Aminlösung werden wäßrige und organische Phase voneinander getrennt. Die organische Phase, sie enthält das Aminsalz, wird mit der Lösung einer Base in Wasser zur Reaktion gebracht, deren sulfonsaures Salz hergestellt werden soll. Beispiele für besonders geeignete Basen sind Natriumhydroxid und Kaliumhydroxid. Man erhält eine wäßrige Lösung, aus dem das gewünschte Sulfonsäuresalz isoliert werden kann.

Statt die in Wasser gelöste Base auf einmal der Lösung des Aminsalzes im organischen Medium zuzusetzen, kann ihre Zugabe auch in Anteilen erfolgen. Eine solche abgestufte Behandlung der Aminlösung, z.B. durch Einstellung bestimmter pH-Werte oder pH-Wertbereiche mit Hilfe der Base ermöglicht eine weitgehende Abtrennung von Phosphinoxiden aus dem Sulfonierungsgemisch und seine partielle Zerlegung in Produkte verschiedener Sulfonierungsstufen.

Die neuen, sulfonierten Diphosphine sind farblose Feststoffe. Je nach Sulfonierungsbedingungen enthalten sie bis zu sechs Sulfonsäuregruppen. Die Alkalisalze sind in Wasser löslich, die Löslichkeit steigt mit dem Sulfonierungsgrad an.

Durch Behandlung wäßriger Alkalisalzlösungen des sulfonierten Diphosphins mit einem Kationaustauscher in der $H^+$-Form können die freien Säuren hergestellt werden. Aus den Säuren lassen sich durch Umsetzung mit Hydroxiden, Carbonaten, Ammoniak oder Aminen weitere Salze der neuen sulfonierten Diphosphine gewinnen.

Die neuen Verbindungen haben sich als Komponenten von Katalysatorsystemen, die Metalle der VIII. Gruppe des Periodensystems enthalten, bewährt. In Kombination mit Rhodium werden sie insbesondere als Hydroformylierungskatalysatoren eingesetzt. Dementsprechend betrifft die Erfindung weiterhin ein Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 20 MPa in Gegenwart wasserlöslicher, Phosphine in komplexer Bindung enthaltender Rhodiumverbindungen als Katalysatoren. Es ist dadurch gekennzeichnet, daß als wasserlösliche Phosphine die vorstehend beschriebenen sulfonierten 2,2'-Bis(diphenylphosphinomethyl)-1,1'-binaphthyle eingesetzt werden.

Die nach dem neuen Verfahren als Katalysator verwendeten wasserlöslichen Rhodium-Diphosphin-Komplexverbindungen zeichnet sich durch eine bemerkenswert hohe Wirksamkeit, bestimmt durch die beiden Kriterien "Aktivität" A, nämlich

$$A = \frac{\text{mol Aldehyd}}{\text{mol Rh min}}$$

und "Produktivität" P, nämlich

$$P = \frac{\text{g Aldehyd}}{\text{ml Katalysatorlösung h}}$$

aus. Die mit den Verfahren des Standes der Technik erreichten Werte für diese beiden Größen werden durch die erfindungsgemäße Arbeitsweise erheblich übertroffen. Die Bildung normaler Aldehyde ist höher und der Austrag von Edelmetall und Phosphin mit dem Reaktionsprodukt ist geringer als bei den bekannten Prozessen. Überdies erzielt man diese Ergebnisse mit einem Katalysator, der ein deutlich geringeres Ligand/Rhodium-Verhältnis aufweist, als die bisher eingesetzten. Diese und andere, für die Durchführung des Prozesses im technischen Maßstab sehr wertvollen Befunde waren weder aus theoretischen Überlegungen ableitbar noch aus der praktischen Erfahrung vorherzusehen.

Es ist nicht erforderlich, die sulfonierten Diphosphine als einheitliche Verbindungen einzusetzen. Auch unterschiedliche Sulfonierungsstufen der Diphosphine und/oder Sulfonatgemische mit verschiedenen Kationen können angewendet werden.

Es hat sich bewährt, Rhodium und die erfindungsgemäßen Diphosphine nicht in stöchiometrischem Verhältnis, also entsprechend der chemischen Zusammensetzung der Rhodium-Komplexverbindung zu verwenden, die sich im Verlauf der Hydroformylierungsreaktion bildet, sondern die Diphosphine im Überschuß einzusetzen. Dabei kann man das Verhältnis von Rhodium und Diphosphin in weiten Grenzen variieren und je mol Rhodium etwa 1 bis 130 mol Diphosphin anwenden. Bevorzugt wird ein Verhältnis von Rhodium zu Diphosphin im Bereich von 1 : 1 bis 1 : 25 und insbesondere 1 : 1 bis 1 : 10 mol.

Rhodium gelangt entweder als Metall oder als Verbindung zum Einsatz. In metallischer Form verwendet man es

in Form feinverteilte Partikel oder in dünner Schicht auf einem Träger wie Aktivkohle, Calciumcarbonat, Aluminiumsilikat, Tonerde, niedergeschlagen. Als Rhodiumverbindungen kommen solche Substanzen in Betracht, die wasserlöslich sind oder unter den Reaktionsbedingungen wasserlöslich werden. Geeignet sind die verschiedenen Rhodiumoxide, Salze anorganischer Wasser- oder Sauerstoffsäuren, sowie Salze aliphatischer Mono- oder Polycarbonsäuren. Beispiele für Rhodiumsalze sind Rhodiumnitrat, Rhodiumsulfat, Rhodiumacetat, Rhodium-2-ethylhexanoat, Rhodiummalonat. Rhodiumhalogenverbindungen sind wegen der geringeren Aktivität der resultierenden Komplexe und wegen des korrosiven Verhaltens der Halogenidionen dagegen weniger brauchbar. Weiterhin können Rhodiumcarbonylverbindungen wie $Rh_3(CO)_{12}$ oder $Rh_6(CO)_{16}$ oder Komplexsalze des Rhodiums, z.B. Cyclooctadienylrhodiumverbindungen eingesetzt werden. Bevorzugt werden Rhodiumoxid und insbesondere Rhodiumacetat und Rhodium-2-ethylhexanoat. Es ist anzunehmen, daß sich in Gegenwart von Synthesegas unter den Bedingungen der Hydroformylierungsreaktion wasserlösliche Rhodium Rhodium-Komplexverbindungen bilden, die Kohlenmonoxid und Diphosphin als Liganden enthalten. Sie ergeben zusammen mit dem im Wasser gelösten Diphosphin-Überschuß das Katalysatorsystem.

Die Katalysatorlösung wird aus den Komponenten entweder im Hydroformylierungsreaktor oder vorab, in einer separaten Vorrichtung hergestellt und darauf dem Hydroformylierungsreaktor zugeleitet.

Die Konzentration des Rhodiums in der wäßrigen Katalysatorlösung beträgt 10 bis 500 Gew.-ppm (bezogen auf die Lösung), vorzugsweise 15 bis 100 Gew.-ppm und insbesondere 10 bis 50 Gew.-ppm.

Die Umsetzung des Olefins mit Kohlenmonoxid und Wasserstoff erfolgt bei Drücken von etwa 0,1 bis etwa 30 MPa, vorzugsweise 1 bis 12 MPa und insbesondere 3 bis 7 MPa. Die Zusammensetzung des Synthesegases, d.h. das Volumenverhältnis von Kohlenmonoxid und Wasserstoff kann sich über weite Bereiche erstrecken und z.B. zwischen 1 : 10 bis 10 : 1 variiert werden. Im allgemeinen setzt man Gasgemische ein, in denen das Volumverhältnis von Kohlenmonoxid und Wasserstoff etwa 1 : 1 ist oder von diesem Wert in der einen oder in der anderen Richtung nur wenig abweicht.

Die Reaktionstemperatur liegt zwischen etwa 20 und 150°C, bevorzugt werden 80 bis 140°C und insbesondere 100 bis 125°C.

Die Umsetzung der in flüssiger und gasförmiger Phase vorliegenden Reaktionspartner erfolgt in konventionellen Reaktoren. Der Ablauf der Umsetzung wird maßgeblich dadurch beeinflußt, daß die wäßrige Katalysatorlösung mit dem flüssigen oder gasförmigen, hydrophoben Olefin und dem Synthesegas gesättigt werden muß. Daher ist es erforderlich, eine möglichst große Berührungsfläche zwischen den Phasen zu erzeugen. Es hat sich bewährt, den flüssigen Reaktorinhalt - Katalysatorlösung, gegebenenfalls flüssiges Olefin und Reaktionsprodukt - intensiv zu rühren und die gasförmigen Reaktionspartner - Synthesegas und gegebenenfalls Olefin - über Verteilungsvorrichtungen der flüssigen Phase zuzuführen. Es hat sich sehr bewährt, den Anteil der organischen Phase im Reaktionsgemisch gering zu halten. Überraschenderweise trägt die organische Phase zur Löslichkeit der Reaktanten in der wäßrigen Phase nicht bei, und es wird vermieden, daß das Reaktionsprodukt unerwünschte Nebenreaktionen eingeht, die bei zunehmender Verweilzeit des Produktes im Reaktor nicht auszuschließen sind. Dementsprechend stellt man ein Volumverhältnis von wäßriger zu organischer Phase von 1 : 1 bis 100 : 1, vorzugsweise 10 : 1 bis 100 : 1 ein. Zu diesem Zweck kann man kontinuierlich einen entsprechenden Teil des Reaktionsgemisches aus dem Reaktor ausschleusen, wäßrige und organische Phase voneinander trennen und die wäßrige Phase in den Reaktor zurückführen. Die Umsetzung kann absatzweise oder, bevorzugt, kontinuierlich durchgeführt werden.

Das erfindungsgemäße Verfahren ist mit Erfolg auf die Umsetzung von Monoolefinen, nicht konjugierten Polyolefinen, cyclischen Olefinen und Derivaten dieser ungesättigten Verbindungen anwendbar. Hinsichtlich der Molekülgröße unterliegen die eingesetzten Olefine keiner Beschränkung, die Arbeitsweise hat sich bei Verbindungen mit 2 bis 40 Kohlenstoffatomen bewährt. Die olefinisch ungesättigten Verbindungen können geradkettig oder verzweigt, die Doppelbindungen end- oder innenständig sein. Beispiele für Olefine, die in dem neuen Prozeß verwendet werden können sind Ethylen, Propylen, Buten-1, Buten-2, Penten-1, 2-Methylbuten-1, Hexen-1, Hexen-2, Hepton-1, Octen-1, Octen-3, 3-Ethylhexen-1, Decen-1, Undecen-3, 4,4-Dimethylnonen-1, Dicyclopentadien, Vinylcyclohexen, Cyclooctadien, Styrol. Derivate der genannten Olefinarten, die nach der beanspruchten Arbeitsweise hydroformyliert werden können, sind z.B. Alkohole, Aldehyde, Carbonsäure, Ester, Nitrile und Halogenverbindungen, Allylakohol, Acrolein, Methacrolein, Crotonaldehyd, Methylacrylat, Ethylcrotonat, Diethylfumarat, Diethylmaleinat, Acrylnitril. Mit besonderem Erfolg wird das Verfahren zur Hydroformylierung von Olefinen und Olefinderivaten mit 2 bis 40, bevorzugt 2 bis 20, und insbesondere 2 bis 8 Kohlenstoffatomen eingesetzt.

In den nachfolgenden Beispielen werden Herstellung und Eigenschaften der neuen Verbindungen (Beispiele 1 bis 10) und ihre Verwendung als Bestandteil von Katalysatoren für die Hydroformylierung olefinisch ungesättigter Verbindungen (Beispiele 12 bis 17) beschrieben.

Beispiel 1

10,11 g (15,54 mmol) 2,2'-Bis(diphenylphosphinomethyl)-1,1'-binaphthalin werden bei Raumtemperatur in 25 ml konzentrierter Schwefelsäure gelöst. Man kühlt die Lösung auf 0°C und versetzt sie, unter Einhaltung einer Höchsttemperatur von 10°C, tropfenweise mit 50 ml 65 %igem Oleum. Darauf wird das Gemisch 48 h bei Raumtemperatur gerührt, anschließend auf Eis gegeben und mit Natronlauge (NaOH-Gehalt: 50 Gew.-%) neutralisiert. Hydrolyse und Neutralisation erfolgen unter solchen Bedingungen, daß eine Temperatur von 25°C nicht überschritten wird. Man filtriert vom ausgefallenen Natriumsulfat ab und rührt das Filtrat in Methanol ein. Der resultierende weiße Feststoff wird abgetrennt und das Filtrat zur Trockene eingeengt. Man nimmt den Rückstand in der zur vollständigen Lösung gerade erforderlichen Menge Wasser auf und spritzt die wäßrige Lösung in das doppelte Volumen Methanol. Die erhaltene Suspension wird abfiltriert, das Filtrat zur Trockene eingeengt. Die vereinigten Filtrate werden analysiert.

Charakterisierung:

Löslichkeit: 1300 g/l Wasser

Elementaranalyse: 13,8 Gew.-% Schwefel; 4,42 Gew.% Phospor; 9,9 Gew.-% Natrium
daraus errechnen sich folgende molare Verhältnisse:
P:S = 1:3; P:Na = 1:3; S:Na = 1:1
entsprechend einer Einführung von sechs $SO_3H$-Gruppen in das 2,2'-Bis(diphenylphosphinomethyl)-1,1'-binaphthalin-Molekül

$^{31}P$-NMR: $\delta = -9{,}0$

Beispiel 2

Die Sulfonierung von 2,2'-Bisldiphenylphosphinomethyl)-1,1'-binaphthalin wird nach Beispiel 1 durchgeführt und der Fortgang der Umsetzung durch NMR-Spektroskopie der schwefelsauren Lösung verfolgt. Folgende Meßwerte werden erhalten:

$^{31}P$-NMR: $\delta$ = - 9,0 (sechsfach sulfoniert)
$\delta$ = - 9,89 (fünffach sulfoniert)
$\delta$ = -12,03 (vierfach sulfoniert)

Beispiele 3 - 10

Die Sulfonierung von 2,2'-Bis(diphenylphosphinomethyl)-1,1'-binaphtalin wird nach Beispiel 1 durchgeführt, jedoch unter Variation der Reaktionsparameter Temperatur, Zeit und $SO_3$-Konzentration. Die Ergebnisse sind in der folgenden Tabelle zusammengestellt. Die Beispiele 5, 6 und 7 sind Vergleichsbeispiele, bei denen höhere Reaktionstemperaturen angewandt wurden.

| Beispiel | $SO_3$-Gehalt [Gew.-%] des Oleums | Reaktionszeit [h] | Temperatur [°C] | Oxidbildung [%][a)] |
|---|---|---|---|---|
| 3 | 20 | 18 | 20 | 5 |
| 4 | 20 | 48 | 20 | 10 |
| 5 | 20 | 48 | 30 | 18 |
| 6 | 20 | 48 | 40 | 52 |
| 7 | 20 | 48 | 50 | 100 |
| 8 | 40 | 48 | 20 | 8 |
| 9 | 65 | 17 | 20 | 5 |
| 10 | 65 | 48 | 20 | 10 |

a) vorherige Standardisierung mit vollständig oxidiertem sechsfach sulfoniertem 2,2'-Bis(diphenylphosphinomethyl)-1,1'-binaphthalin

Beispiel 11

60,7 g (93,2 mmol) 2,2'-Bisldiphenylphosphinomethyl)-1,1'-binaphthalin werden bei 0 bis 10°C in 300 g konzentrierter Schwefelsäure gelöst, unter Einhaltung einer Temperatur von 0 bis 10°C mit 601,7 g 65 %igem Oleum versetzt und darauf 48 h bei Raumtemperatur gerührt. Zur Hydrolyse tropft man das Sulfonierungsgemisch innerhalb von 30 min bei Temperaturen unterhalb 10°C zu 3879,2 g Wasser.

Zur Abtrennung des Sulfonierungsproduktes aus der wäßrigen Phase extrahiert man 1 h bei 40°C mit einer Lösung von 237,4 g Triisooctylamin in 948,8 g Toluol. Anschließend extrahiert man die organische Phase (1307,8 g) bei 40°C

mit 3 Gew.-%iger Natronlauge.

Bis zu einem pH-Wert von 3,5 enthält die wäßrige Phase (357,6 g) 16,8 mmol P(III) und 2,6 mmol P(V), jeweils bezogen auf ein Kilogramm Lösung, sowie 3,8 % Sulfat. Im pH-Bereich 3,5 bis 4,8 beträgt der P(III)-Gehalt der wäßrigen Phase (463,0 g) 163 mmol, der P(V)-Gehalt 15,0 mmol wiederum jeweils bezogen auf ein Kilogramm Lösung und der Sulfatgehalt beträgt 0,05 %. Im Bereich von pH 4,8 bis pH 6,0 wird die Wertproduktfraktion abgetrennt (335,8 g), die, bezogen auf ein Kilogramm Lösung, 212 mmol P(III) und 1,0 mmol P(V) enthält.

Beispiele 12 bis 17

Einem mit Rührer ausgerüsteten 0,2 l Edelstahlautoklav werden Propylen und ein aus gleichen Volumenteilen bestehendes $CO/H_2$-Gemisch in einer solchen Menge zugeleitet, daß 10 Nl/h Abgas aus dem Reaktor entnommen werden können. Gleichzeitig werden je Stunde 300 ml wäßrige Katalysatorlösung (261 mg Rh als Acetat und 13,9 mmol P(III) in Form von sulfoniertem 2,2'-Bis(diphenylphosphinomethyl)-1,1'-binaphthalin (BINAS) gelöst in 1000 ml entgastem und mit Stickstoff gesättigtem Wasser) im Kreis durch den Reaktor geführt. Das Molverhältnis von Phosphor zu Rhodium beträgt 5,5 : 1, entsprchend einem Ligand/Rhodium-Verhältnis von 2,75 : 1. Die Umsetzung der Reaktionspartner erfolgt bei einem Druck von 5 MPa. Die übrigen Reaktionsparameter sind der Tabelle zu entnehmen.

In der Tabelle sind die mit dem erfindungsgemäßen Verfahren erzielten Ergebnisse (Beispiele 12 bis 16) dem Ergebnis gegenübergestellt, das mit einer Arbeitsweise nach dem Stand der Technik [Katalysator : Rhodium/Na-Triphenylphosphintrisulfonat (TPPTS)] erhalten wird (Beispiel 17). Die Versuche machen deutlich, daß nach dem neuen Verfahren bei einem überraschend niedrigen Rh/p-Verhältnis eine hohe Katalysatoraktivität erzielt und das n-/i-Verhältnis weiter gesteigert wird. Auch bei erheblicher Steigerung des Olefineinsatzes (Beispiel 15) erreicht man hohe Umsätze.

Tabelle

| Versuchsbedingungen | Beispiel 12 | Beispiel 13 | Beispiel 14 | Beispiel 15 | Beispiel 16 | Beispiel 17 (Vergleich) |
|---|---|---|---|---|---|---|
| Katalysator | Rh/BINAS | Rh/BINAS | Rh/BINAS | Rh/BINAS | Rh/BINAS | Rh/TPPTS |
| Rhodium/Ligand (mol/mol) | 1:2,75 | 1:2,75 | 1:2,75 | 1:2,75 | 1:2,75 | 1:100 |
| Temperatur (°C) | 110 | 116 | 122 | 122 | 128 | 122 |
| Druck (MPa) | 5,0 | 5,0 | 5,0 | 5,0 | 5,0 | 5,0 |
| Propyleneinsatz (g/h) | 40,0 | 40,0 | 40,0 | 129,0 | 40,0 | 40,0 |
| Versuchsergebnisse | | | | | | |
| Umsatz (%) | 44,7 | 40,4 | 50,8 | 47,9 | 57,8 | 39,0 |
| Aktivität $\frac{\text{mol (n+i) Aldehyd}}{\text{mol Rh . min}}$ | 43,92 | 42,81 | 55,48 | 163,3 | 61,41 | 15,11 |
| Produktivität $\frac{\text{g (n+i) Aldehyd}}{\text{ml Kat.-lsg. . h}}$ | 0,482 | 0,470 | 0,608 | 1,79 | 0,673 | 0,206 |
| n/i-Verhältnis (Gew.-teile) | 97/3 | 97/3 | 97/3 | 98/2 | 97/3 | 93/7 |

**Patentansprüche**

1. Sulfonierte 2,2'-Bis(diphenylphosphinomethyl)-1,1'-binaphthaline der allgemeinen Formel

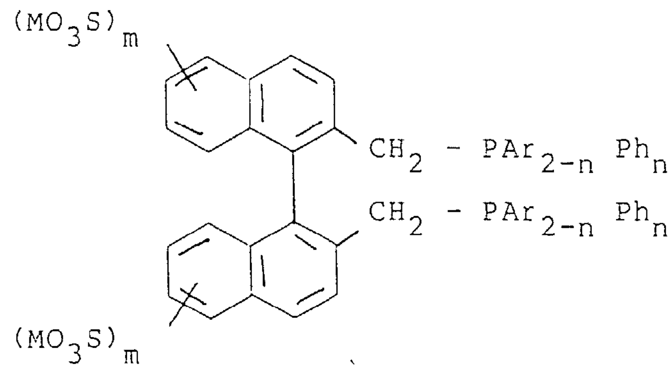

in der Ar für m-$C_6H_4SO_3M$, M für Wasserstoff, für Ammonium, ein einwertiges Metall oder das Äquivalent eines mehrwertigen Metalls und Ph für den Phenylrest steht, m gleich oder verschieden ist und den Wert 1 oder 2 hat und n gleich oder verschieden ist und 0,1 oder 2 bedeutet.

2. Verfahren zur Herstellung der Verbindungen nach Anspruch 1, dadurch gekennzeichnet, daß man 2,2'-Bis(diphenylphosphinomethyl)-1,1'-binaphthaline bei Temperaturen zwischen 0 und 25°C mit Oleum sulfoniert, das Sulfonierungsgemisch hydrolysiert und aus dem hydrolysierten Sulfonierungsgemisch die sulfonierten 2,2'-Bis(diphenylphosphinomethyl)-1,1'-binaphthaline isoliert.

3. Verfahren nach Anspruch 2, dadurch gekennzeichnet, daß man zur Sulfonierung Oleum einsetzt, das 10 bis 65, insbesondere 40 bis 65 Gew.-% freies Schwefeltrioxid enthält.

4. Verfahren nach Anspruch 2 oder 3, dadurch gekennzeichnet, daß man je mol der 2,2'-Bis(diphenylphosphinomethyl)-1,1'-binaphthaline 25 bis 80, vorzugsweise 40 bis 70 mol Schwefeltrioxid einsetzt.

5. Verfahren nach einem oder mehreren der Ansprüche 2 bis 4, dadurch gekennzeichnet, daß die Sulfonierung bei Temperaturen von 0 bis 25°C, vorzugsweise 0 bis 10°C durchgeführt wird.

6. Verfahren nach einem oder mehreren der Ansprüche 2 bis 5, dadurch gekennzeichnet, daß die Hydrolyse des Sulfonierungsgemisches bei Temperaturen bis etwa 30°C, vorzugsweise von 15 bis 25°C vorgenommen wird.

7. Verfahren nach einem oder mehreren der Ansprüche 2 bis 6, dadurch gekennzeichnet, daß man das hydrolysierte Sulfonierungsgemisch mit Alkalicarbonat oder Alkalihydroxid neutralisiert, Alkalisulfat abtrennt, die wäßrige Lösung einengt und die sulfonierten 2,2'-Bis(diphenylphosphinomethyl)-1,1'-binaphthaline aus dem Rückstand mit einem Gemisch aus Alkohlen mit bis zu 5 Kohlenstoffatomen im Molekül und Wasser extrahiert.

8. Verfahren nach einem oder mehreren der Ansprüche 2 bis 6, dadurch gekennzeichnet, daß man das hydrolysierte Sulfonierungsgemisch mit einer Mischung aus einem in Wasser unlöslichen Amin und einem organischen Lösungsmittel, insbesondere einer Mischung aus Triisooctylamin und Toluol umsetzt, die Sulfonate als Aminsalze extrahiert und die Lösung der Aminsalze mit der Lösung einer Base, vorzugsweise Natriumhydroxid oder Kaliumhydroxid in Wasser, gegebenenfalls in Anteilen, versetzt.

9. Verfahren zur Herstellung von Aldehyden durch Umsetzung von Monoolefinen, nichtkonjugierten Polyolefinen, Cycloolefinen oder Derivaten dieser Verbindungsklassen mit Kohlenmonoxid und Wasserstoff bei Temperaturen von 20 bis 150°C und Drücken von 0,1 bis 20 MPa in Gegenwart wasserlöslicher, Diphosphine in komplexer Bindung enthaltender Rhodiumverbindungen als Katalysatoren, dadurch gekennzeichnet, daß als Diphosphine sulfonierte 2,2'-Bis(diphenylphosphinomethyl)-1,1'-binaphthyle der allgemeinen Formel

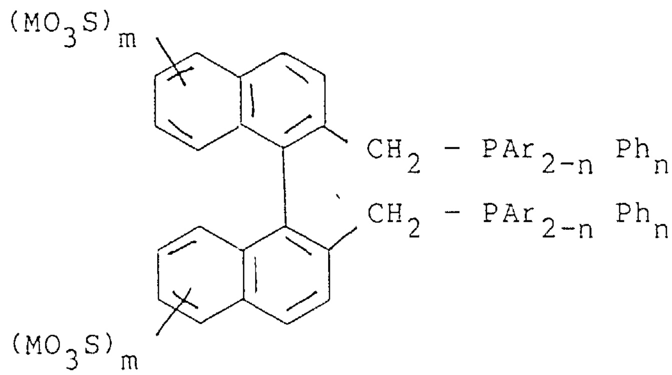

in der Ar für m-$C_6H_4SO_3M$, M für Wasserstoff, für Ammonium, ein einwertiges Metall oder das Äquivalent eines mehrwertigen Metalls und Ph für den Phenylrest steht, m gleich oder verschieden ist und den Wert 1 oder 2 hat und n gleich oder verschieden ist und 0,1 oder 2 bedeutet, eingesetzt werden.

**10.** Verfahren nach Anspruch 9, dadurch gekennzeichnet, daß je mol Rhodium 1 bis 130, bevorzugt 1 bis 25 und insbesondere 1 bis 10 mol sulfonierte Diphosphine eingesetzt werden.

**11.** Verfahren nach Ansprüche 9 oder 10, dadurch gekennzeichnet, daß die Rhodiumkonzentration in der wäßrigen Katalysatorlösung 10 bis 500, vorzugsweise 15 bis 100 und insbesondere 10 bis 50 Gew.-ppm (bezogen auf die Lösung) beträgt.

**12.** Verfahren nach einem oder mehreren der Ansprüche 9 bis 11, dadurch gekennzeichnet, daß die Umsetzung bei 20 bis 150°C, vorzugsweise 80 bis 140°C und insbesondere 100 bis 125°C erfolgt.

**13.** Verfahren nach einem oder mehreren der Ansprüche 9 bis 12, dadurch gekennzeichnet, daß die Umsetzung bei Drücken von 0,1 bis 30, vorzugsweise von 1 bis 12 und insbesondere von 3 bis 7 MPa erfolgt.

**14.** Verfahren nach einem oder mehreren der Ansprüche 9 bis 13, dadurch gekennzeichnet, daß Olefine oder Olefinderivate mit 2 bis 40, vorzugsweise 2 bis 20 und insbesondere 2 bis 8 Kohlenstoffatomen umgesetzt werden.

**15.** Rhodium/Diphosphin-Katalysator zur Hydroformylierung von Monoolefinen, nichtkonjugierten Polyolefinen, Cycloolefinen oder Derivaten dieser Verbindungsklassen, dadurch gekennzeichnet, daß er je mol Rhodium 1 bis 130, vorzugsweise 1 bis 25 und insbesondere 1 bis 10 mol sulfoniertes 2,2'-Bis(diphenylphosphinomethyl)-1,1'-binaphthyl enthält.

## Claims

**1.** A sulfonated 2,2'-bis(diphenylphosphinomethyl)-1,1'-binaphthalene of the formula

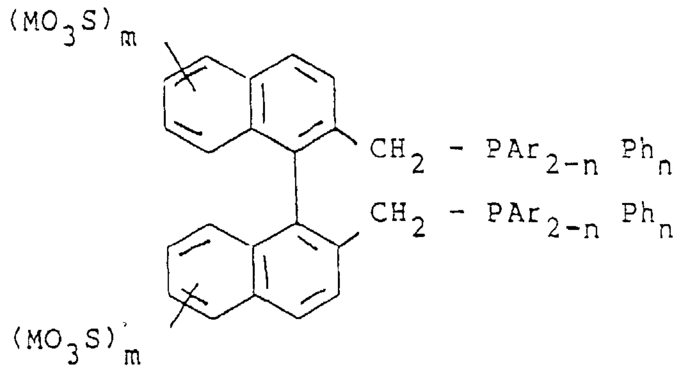

in which Ar is m-$C_6H_4SO_3M$, M is hydrogen, ammonium, a monovalent metal or the equivalent of a polyvalent metal, Ph is the phenyl radical, m is identical or different and has the value 1 or 2 and n is identical or different and is 0, 1 or 2.

2. A process for the preparation of the compounds as claimed in claim 1, which comprises sulfonating 2,2'-bis(diphenylphosphinomethyl)-1,1'-binaphthalenes with oleum at temperatures of between 0 and 25°C, hydrolyzing the sulfonation mixture and isolating the sulfonated 2,2'-bis(diphenylphosphinomethyl)-1,1'-binaphthalenes from the hydrolyzed sulfonation mixture.

3. The process as claimed in claim 2, wherein oleum containing 10 to 65 and especially 40 to 65% by weight of free sulfur trioxide is used for the sulfonation.

4. The process as claimed in claim 2 or 3, wherein 25 to 80 and preferably 40 to 70 mol of sulfur trioxide are used per mole of the 2,2'-bis(diphenylphosphinomethyl)-1,1'-binaphthalenes.

5. The process as claimed in one or more of claims 2 to 4, wherein the sulfonation is carried out at temperatures of 0 to 25°C, preferably 0 to 10°C.

6. The process as claimed in one or more of claims 2 to 5, wherein the hydrolysis of the sulfonation mixture is carried out at temperatures of up to about 30°C, preferably of 15 to 25°C.

7. The process as claimed in one or more of claims 2 to 6, wherein the hydrolyzed sulfonation mixture is neutralized with alkali metal carbonate or alkali metal hydroxide, alkali metal sulfate is separated off, the aqueous solution is concentrated and the sulfonated 2,2'-bis(diphenylphosphinomethyl)-1,1'-binaphthalenes are extracted from the residue with a mixture of alcohols having up to 5 carbon atoms in the molecule and water.

8. The process as claimed in one or more of claims 2 to 6, wherein the hydrolyzed sulfonation mixture is reacted with a mixture of a water-insoluble amine and an organic solvent, especially a mixture of triisooctylamine and toluene, the sulfonates are extracted as amine salts and the aqueous solution of a base, preferably sodium hydroxide or potassium hydroxide, is added to the solution of the amine salts, optionally in portions.

9. A process for the preparation of aldehydes by reaction of monoolefins, nonconjugated polyolefins, cycloolefins or derivatives of these classes of compounds with carbon monoxide and hydrogen at temperatures of 20 to 150°C and pressures of 0.1 to 20 MPa in the presence of catalysts composed of watersoluble compounds of rhodium complexed with diphosphines, wherein the diphosphines used are sulfonated 2,2'-bis(diphenylphosphinomethyl)-1,1'-binaphthyls of the formula

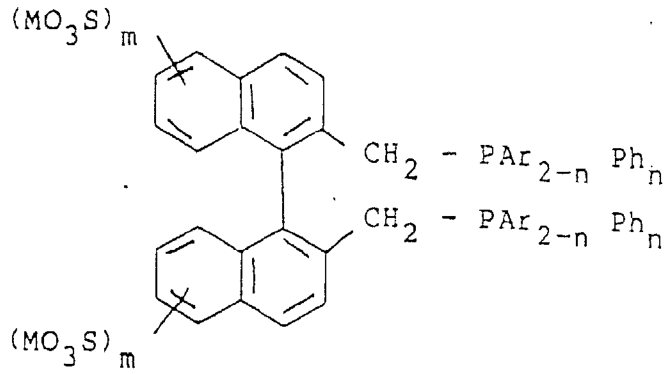

in which Ar is m-$C_6H_4SO_3M$, M is hydrogen, ammonium, a monovalent metal or the equivalent of a polyvalent metal, Ph is the phenyl radical, m is identical or different and has the value 1 or 2 and n is identical or different and is 0, 1 or 2.

**10.** The process as claimed in claim 9, wherein 1 to 130, preferably 1 to 25 and especially 1 to 10 mol of sulfonated diphosphines are used per mole of rhodium.

**11.** The process as claimed in claim 9 or 10, wherein the rhodium concentration in the aqueous catalyst solution is 10 to 500, preferably 15 to 100 and especially 10 to 50 ppm by weight (based on the solution).

**12.** The process as claimed in one or more of claims 9 to 11, wherein the reaction is carried out at 20 to 150°C, preferably 80 to 140°C and especially 100 to 125°C.

**13.** The process as claimed in one or more of claims 9 to 12, wherein the reaction is carried out at pressures of 0.1 to 30, preferably 1 to 12 and especially 3 to 7 MPa.

**14.** The process as claimed in one or more of claims 9 to 13, wherein olefins or olefin derivatives having 2 to 40, preferably 2 to 20 and especially 2 to 8 carbon atoms are reacted.

**15.** A rhodium/diphosphine catalyst for the hydroformylation of monoolefins, nonconjugated polyolefins, cycloolefins or derivatives of these classes of compounds, which contains 1 to 130, preferably 1 to 25 and especially 1 to 10 mol of sulfonated 2,2'-bis(diphenylphosphinomethyl)-1,1'-binaphthyl per mole of rhodium.

## Revendications

**1.** 2,2'-bis(diphénylphosphinométhyl)-1,1'-binaphtalènes sulfonés de formule générale

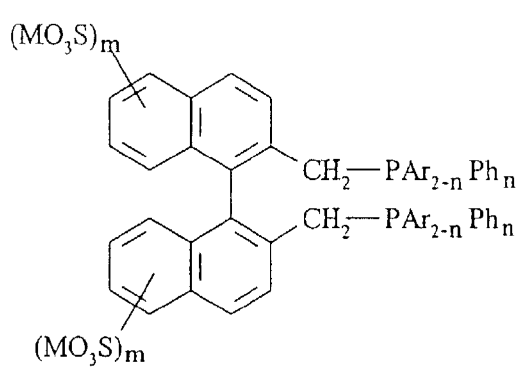

dans laquelle Ar représente le groupe m-$C_6H_4$-$SO_3M$, M représente un atome d'hydrogène, un groupe ammonium,

un métal monovalent ou l'équivalent d'un métal multivalent et Ph représente le reste phényle, les m sont identiques ou différents et ont la valeur 1 ou 2, et les n sont identiques ou différents et ont la valeur 0, 1 ou 2.

**2.** Procédé de préparation des composés selon la revendication 1, caractérisé en ce que l'on effectue une sulfonation de 2,2'-bis(diphénylphosphinométhyl)-1,1'-binaphtalènes avec de l'oléum à des températures comprises entre 0 et 25°C, on hydrolyse le mélange de sulfonation et on isole les 2,2'-bis(diphénylphosphinométhyl)-1,1'-binaphtalènes sulfonés à partir du mélange de sulfonation hydrolysé.

**3.** Procédé selon la revendication 2, caractérisé en ce que l'on utilise pour la sulfonation un oléum qui contient 10 à 65 %, en particulier 40 à 65 % en masse de trioxyde de soufre libre.

**4.** Procédé selon la revendication 2 ou 3, caractérisé en ce que, pour une mole de 2,2'-bis(diphénylphosphinométhyl)-1,1'-binaphtalène, on utilise 25 à 80 mol, de préférence 40 à 70 mol de trioxyde de soufre.

**5.** Procédé selon l'une ou plusieurs des revendications 2 à 4, caractérisé en ce que l'on effectue la sulfonation à des températures de 0 à 25°C, de préférence de 0 à 10°C.

**6.** Procédé selon l'une ou plusieurs des revendications 2 à 5, caractérisé en ce que l'hydrolyse du mélange de sulfonation s'effectue à des températures allant jusqu'à environ 30°C, de préférence de 15 à 25°C.

**7.** Procédé selon l'une ou plusieurs des revendications 2 à 6, caractérisé en ce que l'on neutralise le mélange de sulfonation hydrolysé avec un carbonate de métal alcalin ou un hydroxyde de métal alcalin, on sépare le sulfate de métal alcalin, on concentre la solution aqueuse et on extrait les 2,2'-bis(diphénylphosphinométhyl)-1,1'-binaphtalènes sulfonés du résidu avec un mélange d'alcools ayant jusqu'à 5 atomes de carbone dans la molécule et d'eau.

**8.** Procédé selon l'une ou plusieurs des revendications 2 à 6, caractérisé en ce que l'on fait réagir le mélange de sulfonation hydrolysé avec un mélange d'une amine insoluble dans l'eau et d'un solvant organique, en particulier un mélange de triisooctylamine et de toluène, on extrait les sulfonates sous forme de sels d'amine et on ajoute à la solution des sels d'amine une solution d'une base, de préférence d'hydroxyde de sodium ou d'hydroxyde de potassium, dans de l'eau, éventuellement par portions.

**9.** Procédé de préparation d'aldéhydes par réaction de monooléfines, de polyoléfines non conjuguées, de cyclooléfines ou de dérivés de cette classe de composés, avec du monoxyde de carbone et de l'hydrogène à des températures de 20 à 150°C et sous des pressions de 0,1 à 20 MPa, en présence de composés de rhodium solubles dans l'eau contenant des diphosphines en liaison complexe comme catalyseurs, caractérisé en ce que en ce que l'on utilise comme diphosphines des 2,2'-bis(diphénylphosphinométhyl)-1,1'-binaphtalènes sulfonés de formule générale

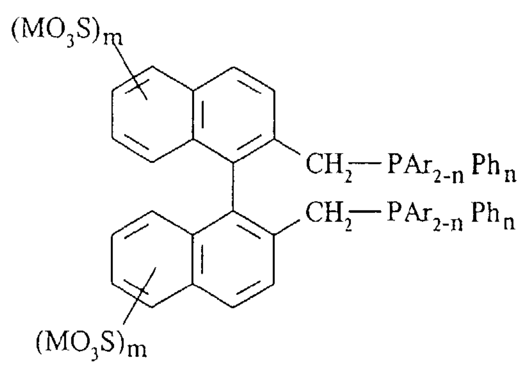

dans laquelle Ar représente le groupe m-$C_6H_4$-$SO_3M$, M représente un atome d'hydrogène, un groupe ammonium, un métal monovalent ou l'équivalent d'un métal multivalent et Ph représente le reste phényle, les m sont identiques ou différents et ont la valeur 1 ou 2, et les n sont identiques ou différents et ont la valeur 0, 1 ou 2.

**10.** Procédé selon la revendication 9, caractérisé en ce que l'on utilise 1 à 130 mol, de préférence 1 à 25 mol et en

particulier 1 à 10 mol de diphosphines sulfonées par mol de rhodium.

11. Procédé selon les revendications 9 ou 10, caractérisé en ce que la concentration de rhodium dans la solution aqueuse de catalyseur est de 10 à 500, de préférence de 15 à 100 et en particulier de 10 à 50 ppm en masse (par rapport à la solution).

12. Procédé selon l'une ou plusieurs des revendications 9 à 11, caractérisé en ce que la réaction s'effectue à une température de 20 à 150°C, de préférence de 80 à 140°C et en particulier de 100 à 125°C.

13. Procédé selon l'une ou plusieurs des revendications 9 à 12, caractérisé en ce que la réaction s'effectue sous des pressions de 0,1 à 30, de préférence de 1 à 12 et en particulier de 3 à 7 MPa.

14. Procédé selon l'une ou plusieurs des revendications 9 à 13, caractérisé en ce qu'on met à réagir des oléfines ou des dérivés d'oléfines de 2 à 40, de préférence 2 à 20 et en particulier 2 à 8 atomes de carbone.

15. Catalyseur de rhodium-diphosphine pour l'hydroformylation de monooléfines, de polyoléfines non conjuguées, de cyclooléfines ou de dérivés de ces classes de composés, caractérisé en ce qu'il contient 1 à 130, de préférence 1 à 25 et en particulier 1 à 10 mol de 2,2'-bis(diphénylphosphinométhyl)-1,1'-binaphtalène par mol de rhodium.